# EUROPEAN PATENT SPECIFICATION

(11) **EP 3 597 077 B1**
(45) Date of publication and mention of the grant of the patent: **29.09.2021**
(21) Application number: 18767132.6
(22) Date of filing: 12.02.2018
(51) Int. Cl.: A47C 27/14, A47D 13/08, A61G 13/00, A47D 15/00

(54) **MEDICAL EXAMINATION TABLE FOR BABIES**
MEDIZINISCHER UNTERSUCHUNGSTISCH FÜR BABYS
CIVIÈRE POUR LES INTERVENTIONS SUR LES BÉBÉS

(30) Priority: 15.03.2017 ES 201730269 U
(43) Date of publication of application: 22.01.2020
(73) Proprietor: Fama Sofas, S.L., 30510 Yecla (Murcia) (ES)
(72) Inventor: LÓPEZ MARTÍNEZ, Paloma, 30510 Yecla (Murcia) (ES); LÓPEZ GIL, Félix, 30510 Yecla (Murcia) (ES)
(74) Representative: Ballester Cañizares, Rosalia
(86) International application number: PCT/ES2018/070097
(87) International publication number: WO 2018/167339

(56) References cited:
- WO-A1-2007/119922
- FR-A1- 3 040 129
- GB-A- 2 498 718
- US-A- 4 790 041
- US-A1- 2004 261 668
- US-A1- 2005 278 854
- US-A1- 2009 045 233
- US-B1- 9 486 072

## Description

### OBJECT OF THE INVENTION

The present invention relates to a table for any type of minor outpatient and hospital medical examinations for babies, such as examinations of the eyes, nose, or mouth, as well as physical therapy and rehabilitation treatments for babies in the body and limbs.

### BACKGROUND OF THE INVENTION

Today there are minor outpatient-type medical examinations for babies that can be performed, for example, in the fields of otorhinolaryngology or dentistry, that do not offer suitable support for the babies, because the medical examination seats or chairs in these specialty fields and other similar ones are conceived for adults and even children, such that they can be adjusted in height, but not for babies who cannot sit upright or who may make involuntary movements during the medical examination.

These drawbacks are solved with the use of the table of the invention.

### DESCRIPTION OF THE INVENTION

The table of the invention has a configuration which allows placing the baby comfortably on the legs of the doctor during a medical examination, therefore in an optimal and up-close position, and with a simple and economical configuration.

According to the invention, the table comprises a body having an upper surface adapted anatomically to receive a baby lying down, and a lower face comprising a projection adapted to the legs of the doctor. This projection thereby fits on the legs of the doctor, ensuring perfect seating of the table, while the upper part is used for receiving the baby lying down.

The following documents are examples of relevant prior art for the present invention: US 2004/261668 A1, US 2009/045233 A1, WO 2007/119922 A1 and US 9486072 B1.

### DESCRIPTION OF THE DRAWINGS

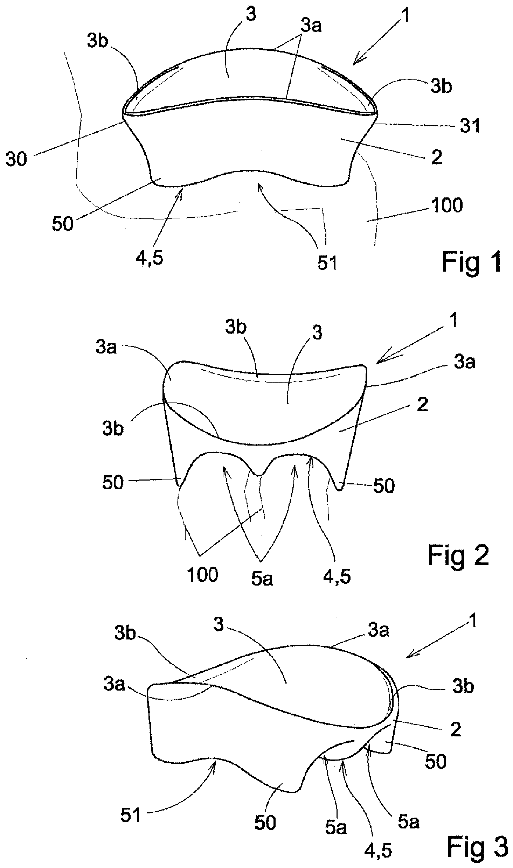

Figures 1 to 3 show respective views of the table of the invention from different angles.

### PREFERRED EMBODIMENT OF THE INVENTION

The medical examination table (1) for babies of the invention comprises a body (2) having an upper surface (3) adapted anatomically to receive a baby lying down, and a lower face (4) comprising a projection (5) adapted to the legs (100) of the doctor.

The upper surface (3) comprises an upwardly oriented concave cross-section, which allows the baby to be protected and held, preventing voluntary or involuntary lateral movements and slipping out. Furthermore, the upper surface (3) comprises central areas (3a) with a greater height on both sides, because greater protection is required in this area as it comprises the baby's torso, which has a higher inertia for slipping out because it weighs more, leaving more space at the end zones corresponding to the head or lower limbs so that the doctor can work comfortably. It has also been envisaged that the upper surface (3) preferably comprises ascending edges (3b) at the proximal end (30) and distal end (31) thereof for holding a cushion, not depicted.

In turn, the projection (5) adapted to the legs of the doctor of the lower face (5) of the body (2) ideally comprises a cross-section with two downwardly oriented adjacent depressions (5a) in which both legs (100) of the doctor fit. Preferably, the outer sides (50) of the two depressions (5a) comprise in the central part thereof an area (51) having a smaller height than at the end parts thereof to increase the doctor's comfort.

In the preferred configuration, the body (2) has a single-piece configuration and allows being manufactured from plastics.

Having sufficiently described the nature of the invention as well as the manner of carrying it out to practice, it must be stated that the arrangements indicated above and depicted in the attached drawings are susceptible to modifications in detail provided that they do not alter the principle thereof as determined in the set of claims, said claims defining the subject matter for which protection is sought.

## Claims

1. A medical examination table (1) for babies, comprising a body (2) having an upper surface (3) adapted anatomically to receive a baby lying down, and a lower face (4) comprising a projection (5) adapted to the legs (100) of the doctor, said projection (5) comprising a cross-section with two downwardly oriented adjacent depressions (5a); **characterised in that** the outer sides (50) of the two depressions (5a) comprise in the central part thereof an area (51) having a smaller height than at the end parts thereof.

2. The medical examination table (1) for babies according to claim 1, **characterised in that** the upper surface (3) comprises an upwardly oriented concave cross-section.

3. The medical examination table (1) for babies according to claim 2, **characterised in that** the upper surface (3) comprises central areas (3a) with a greater height on both sides.

4. The medical examination table (1) for babies according to any of the preceding claims, **characterised in that** the upper surface (3) comprises ascending edges (3b) at the proximal end (30) and distal end (31) thereof for holding a cushion.

5. The medical examination table (1) for babies according to any of the preceding claims, **characterised in that** the body (2) has a single-piece configuration.

6. The medical examination table (1) for babies according to any of the preceding claims, **characterised in that** the body (2) is made from plastics.

## Patentansprüche

1. Medizinischer Untersuchungstisch (1) für Säuglinge, umfassend einen Körper (2) mit einer oberen Oberfläche (3), die anatomisch an die Aufnahme eines liegenden Babys angepasst ist, und eine Unterseite (4) umfassend einen Vorsprung (5), der an die Beine (100) des Schabers angepasst ist, wobei der Vorsprung (5) einen Querschnitt mit zwei nach unten gerichteten, benachbarten Vertiefungen (5a) aufweist; **dadurch gekennzeichnet, dass** die Außenseiten (50) der beiden Vertiefungen (5a) in ihrem mittleren Teil einen Bereich (51) aufweisen, der eine geringere Höhe als an ihren Endteilen hat.

2. Medizinischer Untersuchungstisch (1) für Säuglinge nach Anspruch 1, **dadurch gekennzeichnet, dass** die obere Fläche (3) einen nach oben gerichteten konkaven Querschnitt aufweist.

3. Medizinischer Untersuchungstisch (1) für Säuglinge nach Anspruch 2, **dadurch gekennzeichnet, dass** die obere Fläche (3) beidseitig mittlere Bereiche (3a) mit größerer Höhe aufweist.

4. Medizinischer Untersuchungstisch (1) für Säuglinge nach einem der vorhergehenden Ansprüche, **dadurch gekennzeichnet, dass** die obere Fläche (3) an ihrem proximalen Ende (30) und distalen Ende (31) ansteigende Kanten (3b) zur Aufnahme eines Kissens aufweist.

5. Medizinischer Untersuchungstisch (1) für Säuglinge nach einem der vorhergehenden Ansprüche, **dadurch gekennzeichnet, dass** der Körper (2) einteilig ausgebildet ist.

6. Medizinischer Untersuchungstisch (1) für Säuglinge nach einem der vorhergehenden Ansprüche, **dadurch gekennzeichnet, dass** der Korpus (2) aus Kunststoff gefertigt ist.

## Revendications

1. Table d'examen médical (1) pour bébés, comprenant une structure (2) ayant une surface supérieure (3) adaptée de manière anatomique pour recevoir un bébé allongé, et une face inférieure (4) comprenant une saillie (5) adaptée aux jambes (100) du médecin, ladite saillie (5) comprenant une section transversale avec deux dépressions (5a) adjacentes orientées vers le bas ; **caractérisé par le fait que** les côtés extérieurs (50) des deux dépressions (5a) comprennent dans leur partie centrale une zone (51) ayant une hauteur inférieure à celle de leurs parties d'extrémité.

2. La table d'examen médical (1) pour bébés selon la revendication 1, **caractérisé par le fait que** la surface supérieure (3) comprend une section transversale concave orientée vers le haut.

3. La table d'examen médical (1) pour bébés selon la revendication 2, **caractérisé par le fait que** la surface supérieure (3) comprend des zones centrales (3a) avec une plus grande hauteur des deux côtés.

4. La table d'examen médical (1) pour bébés selon l'une quelconque des revendications précédentes, **caractérisé par le fait que** la surface supérieure (3) comprend des bords ascendants (3b) à son extrémité proximale (30) et à son extrémité distale (31) pour maintenir un coussin.

5. La table d'examen médical (1) pour bébés selon l'une quelconque des revendications précédentes, **caractérisé par le fait que** la structure (2) a une configuration monobloc.

6. La table d'examen médical (1) pour bébés selon l'une quelconque des revendications précédentes, **caractérisé par le fait que** la structure (2) est fabriquée en matière plastique.
